Europäisches Patentamt

European Patent Office

Office européen des brevets

(11) Numéro de publication : **0 403 325 B1**

(12) ## FASCICULE DE BREVET EUROPEEN

(45) Date de publication du fascicule du brevet :
**20.07.94 Bulletin 94/29**

(51) Int. Cl.$^5$ : **C07C 59/60**

(21) Numéro de dépôt : **90401409.9**

(22) Date de dépôt : **28.05.90**

(54) **Acide diallyloxyacétique et ses sels d'addition basiques, leur procédé de préparation et leur application comme agent réticulant.**

(30) Priorité : **14.06.89 FR 8907894**

(43) Date de publication de la demande :
**19.12.90 Bulletin 90/51**

(45) Mention de la délivrance du brevet :
**20.07.94 Bulletin 94/29**

(84) Etats contractants désignés :
**AT BE CH DE DK ES FR GB GR IT LI LU NL SE**

(56) Documents cités :
**EP-A- 0 115 007**

(73) Titulaire : **SOCIETE FRANCAISE HOECHST
TOUR ROUSSEL HOECHST
1 Terrasse Bellini
F-92800 Puteaux (FR)**

(72) Inventeur : **Mallo, Paul
290 avenue Napoléon Bonaparte
F-92500 Rueil Malmaison (FR)**
Inventeur : **Sidot, Christian
Résidence des Fleurs,
avenue Foch
F-95460 Ezanville (FR)**
Inventeur : **Blanc, Alain
61 Avenue Romain Rolland
F-93200 Saint Denis (FR)**
Inventeur : **Christidis, Yani
53 boulevard de la Villette
F-75019 Paris (FR)**

(74) Mandataire : **Rinuy, Santarelli
14, avenue de la Grande Armée
F-75017 Paris (FR)**

## Description

La présente invention concerne l'acide diallyloxyacétique et ses sels d'addition basiques, leur procédé de préparation et leur application comme agent réticulant.

Les agents réticulants sont couramment utilisés pour accéder à des polymères présentant des propriétés particulières ou améliorées. Ils sont généralement employés à faibles doses et l'on souhaite qu'ils conduisent à des polymères réticulés homogènes. Ils doivent donc posséder des propriétés adéquates de solubilité, de réactivité, de compatibilité envers les monomères ou les polymères avec lesquels on désire les faire réagir.

On sait que l'on obtient aisément des polymères réticulés par polymérisation radicalaire, notamment en solution ou en suspension, d'un ou plusieurs monomères éthyléniques en présence d'un agent réticulant convenable, comportant au moins deux fonctions éthyléniques polymérisables tel que les composés di- ou polyvinyliques, les di- ou polyesters d'acides mono- ou polycarboxyliques éthyléniques avec des polyols, les bisacrylamides comme le N,N-méthylène bisacrylamide ou l'acide bisacrylamidoacétique. Parmi tous ces agents réticulants polyéthyléniques connus, l'acide bisacrylamidoacétique salifié est, à la connaissance de la Demanderesse, le seul qui soit très soluble dans l'eau mais c'est un dérivé de l'acrylamide dont on souhaite l'absence pour certains usages particuliers en raison de sa toxicité.

On recherche donc un agent réticulant diéthylénique, hydrosoluble, compatible notamment avec l'acide acrylique ou ses sels d'ammonium ou de métaux alcalins, ne possédant pas dans sa structure de fragment acrylamide, pour la fabrication de polymères réticulés, insolubles dans l'eau mais gonflables à l'eau tels que des polymères épaississants ou super-absorbants destinés notamment à des usages cosmétiques et/ou hygiéniques, comme la fabrication des couches pour bébés.

Le diallyloxyacétate de sodium a déjà été préparé par L. Holmquist, Acta Chem. Scand., 1969, 23, 1045-52, par condensation de l'acide dichloracétique, sur un excès de propène-2 olate-1 de sodium, afin de tester son activité inhibitrice éventuelle sur le neuraminidase du vibrio cholerae. Le diallyloxyacétate d'allyle est par ailleurs décrit dans le brevet des Etats-Unis d'Amérique N° 4.454.065 et dans la demande de brevet européen N°0.094.815. Dans ces documents, il est mentionné comme présentant un point d'ébullition de 55-60°C sous 16 mm/Hg très différent de celui trouvé par la Demanderesse.

La Demanderesse a découvert avec étonnement de nouveaux agents réticulants hydrosolubles éthyléniques répondant aux besoins des utilisateurs et ne présentant pas les inconvénients de ceux de l'art antérieur.

C'est pourquoi la présente demande a pour objet l'acide diallyloxyacétique et ses sels d'addition avec les bases minérales, à l'exception du sel de sodium.

Parmi ces produits, on peut citer plus particulièrement :
- l'acide diallyloxyacétique ;
- le diallyloxyacétate de potassium ;
- le diallyloxyacétate d'ammonium.

On peut citer également le diallyloxyacétate de lithium.

Selon l'invention, les produits de formule générale (I) ci-dessus peuvent être préparés par un procédé caractérisé en ce que l'on effectue une hydrolyse alcaline du diallyloxyacétate d'allyle pour obtenir le sel basique correspondant que soit l'on isole, soit si désire l'on acidifie en acide diallyloxyacétique que l'on isole ou si désiré, salifie en sel d'ammonium.

Dans des conditions préférentielles de mise en oeuvre, le procédé ci-dessus est caractérisé en ce que l'on fait réagir l'acide glyoxylique avec l'alcool allylique pour obtenir le diallyloxyacétate d'allyle.

L'on fait réagir l'acide glyoxylique de préférence avec un excès d'alcool allylique en présence d'un catalyseur acide et, éventuellement, d'un solvant non miscible à l'eau donnant un azéotrope avec l'eau, pour obtenir le diallyloxyacétate d'allyle.

L'hydrolyse est réalisée en faisant réagir le diallyloxyacétate d'allyle avec l'hydroxyde de métal alcalin correspondant. Si désiré, l'on acidifie les sels basiques en acide diallyloxyacétique, de préférence a l'aide d'un acide organique dont le sel correspondant est peu soluble dans l'eau.

Dans des conditions préférentielles de mise en oeuvre de l'invention, le procédé ci-dessus décrit est réalisé de la manière suivante :

1. La réaction de l'alcool allylique avec l'acide glyoxylique est réalisée :
- avec un excès de 5 à 12 moles, avantageusement avec 10 moles d'alcool par mole d'acide glyoxylique,
- en présence d'un solvant choisi parmi le toluène, le cyclohexane et le trichloro-1,1,1-éthane, en présence d'un catalyseur acide choisi parmi l'acide paratoluènesulfonique, l'acide sulfurique et les résines sulfoniques, échangeuses d'ions, sous forme acide,
- avec distillation azéotropique concomitante de l'eau présente et formée dans le milieu réactionnel.

2. L'hydrolyse du diallyloxyacétate d'allyle isolé au premier stade du procédé par des moyens connus en soi est réalisée :
- à la température ambiante avec un léger défaut d'hydroxyde de sodium, d'hydroxyde de potassium ou d'hydroxyde de lithium,
- avec élimination de l'alcool allylique libéré par distillation azéotropique avec de l'eau.

3. L'acidification du diallyloxyacétate de sodium, de potassium ou de lithium en acide diallyloxyacétique est réalisée :
- à la température ambiante, dans un milieu biphasique eau-solvant organique non miscible à l'eau,
- avec un léger défaut d'acide oxalique.

4. Le diallyloxyacétate d'ammonium est obtenu en salifiant l'acide diallyloxyacétique avec de l'ammoniac, soit sous forme liquide aux environs de -30°C, soit en solution dans un solvant organique soit dans l'eau, puis en évaporant sous vide le milieu réactionnel obtenu.

La présente invention a également pour objet l'utilisation des produits de formule générale (I) comme agent de réticulation. En effet, ces produits peuvent être aisément copolymérisés, même à très faibles doses, avec divers monomères éthyléniques pour fournir des copolymères réticulés possédant des propriétés particulières ou améliorées. Ils peuvent être utilisés comme comonomère aussi bien dans des procédés de polymérisation en solution, en suspension ou en émulsion eau dans huile ou huile dans eau, avec des monomères tels que l'acide acrylique, l'acrylate de sodium, l'acrylate de potassium, l'acrylate d'ammonium, l'acrylate de diméthylaminoéthyle libre, salifié ou quaternisé, l'acrylamide, et les dérivés similaires correspondant à l'acide méthacrylique, le vinylsulfonate de sodium, le chlorure de diallyldiméthyl-ammonium, l'acide (méth)acrylamido-2 méthyl-2 propanesulfonique-1, l'acide hydroxy-2 (méth)acryloyloxy-3 propanesulfonique-1, l'acide itaconique, l'acide citraconique, l'acide moléique. Parmi tous les monomères éthyléniques ci-dessus, les monomères préférés sont l'acide acrylique, les acrylates de métal alcalin, et l'acrylate d'ammonium.

Les produits de formule générale (I) sont particulièrement intéressants pour l'obtention de polymères hydrophiles insolubles dans l'eau mais gonflables à l'eau tels que les polymères épaississants, les polymères super-absorbants, à base d'acide acrylique libre, partiellement ou totalement salifié. En effet, avec ces monomères, les produits de formule générale (I) se copolymérisent aisément et d'une manière homogène, même à des doses pouvant atteindre 0,001 % en proportions molaires, pour fournir des polymères réticulés quasiment insolubles dans l'eau c'est-à-dire présentant un taux d'extractibles inférieur à 10 % en poids par rapport au poids de polymère sec, et présentant par ailleurs d'excellentes propriétés absorbantes.

Les exemples suivants illustrent l'invention sans toutefois la limiter. Dans ces exemples, les tests utilisés ont été réalisés de la manière suivante :
- la capacité d'absorption d'eau du polymère est déterminée à 20°C, en agitant durant 30 minutes, 0,4 g de polymère dans 500 g d'eau, puis en pesant le gel polymère obtenu égoutté. Le poids trouvé est ramené à 1 g de polymère ;
- la capacité d'absorption de solution aqueuse saline du polymère est déterminée à 20°C, en agitant durant 30 minutes, 2 g de polymère dans 500 g d'une solution aqueuse contenant 9 g/l de chlorure de sodium puis en pesant le gel polymère obtenu égoutté. Le poids trouvé est ramené comme précédemment à 1 g de polymère .

Le taux d'extractibles est déterminé selon la méthode suivante :
- on place 1 g de polymère à tester dans 200 g d'une solution aqueuse contenant 9 g/l de chlorure de sodium ;
- on agite cette suspension pendant 16 heures à 20°C ;
- on égoutte le gel polymère obtenu et on recueille le filtrat ;
- on dose sur 100 cm³ du filtrat les fonctions carboxyliques et carboxylates présentes ;
- on exprime le résultat de ce dosage par gramme de polymère dissous pour 100 g de polymère sec.

## Exemple 1

On chauffe au reflux avec distillation azéotropique de l'eau et recyclage du solvant d'entraînement, un mélange constitué par :
- 92,6 g d'acide glyoxylique à 80 en poids dans l'eau, soit 1 mole,
- 581 g (10 moles) d'alcool allylique,
- 110 g de trichloro-1,1,1-éthane,
- 2,5 g d'acide sulfurique concentré à 98 %.

Après 330 minutes de chauffage, on a recueilli la quantité d'eau théorique, on refroidit alors le milieu réactionnel puis on le neutralise avec de l'hydrogénocarbonate de sodium et on filtre ensuite les sels minéraux obtenus. Le filtrat est ensuite concentré sous vide pour éliminer l'excès d'alcool allylique et le solvant, puis l'huile résiduelle est distillée. On recueille ainsi 50,7 g (0,29 mole) d'allyloxy-2 glycolate d'allyle, distillant à 50°C sous 5mbar puis 148,6 g (0,7 mole) de diallyloxyacétate d'allyle à 108°C sous 5mbar.

L'allyloxy-2 glycolate d'allyle est cité sans constante physique dans le brevet européen N° 0.109.044.

## Exemple 2

### Préparation du diallyloxyacétate de sodium

On dissout 21,2 g (0,1 mole) de diallyloxyacétate d'allyle dans 42 g de toluène puis on introduit dans cette solution 3,92 g (98 mmoles) d'hydroxyde de sodium dissous dans 50 g d'eau. On agite vigoureusement ce milieu réactionnel biphasique pendant 30 minutes à 30°C, puis on décante et l'on recueille la phase aqueuse que l'on soumet à une distillation sous vide pour éliminer par distillation azéotropique l'alcool allylique libéré. La phase aqueuse est ensuite versée dans de l'acétone pour précipiter le diallyloxyacétate de sodium attendu.

On filtre, puis le précipité obtenu est séché sous vide à poids contant à 60°C. On obtient ainsi 19g (98 mmoles) de diallyloxyacétate de sodium cristallisé pur présentant un point de fusion de 179-181°C (littérature citée F 179-181°C).

Ce produit est très soluble dans l'eau.

## Exemple 3

### Préparation de l'acide diallyloxyacétique

Dans une solution aqueuse contenant 29,1 g (0,15 mole) de diallyloxyacétate de sodium et 71 g d'eau, on introduit à la température ambiante et sous agitation 100 g d'oxyde de diéthyle puis une solution de 17,6 g (0,14 mole) d'acide oxalique cristallisé avec deux molécules d'eau dans 62 g d'eau. Le milieu biphasique obtenu est agité vigoureusement pendant 10 minutes puis il est décanté. La phase éthérée est ensuite séchée sur sulfate de sodium anhydre, filtrée et enfin concentrée à sec sous vide. On obtient ainsi 24 g (0,14 mole) d'acide diallyloxyacétique pur. Cet acide se présente sous la forme d'une huile, il possède un pka de 2.9 et il est soluble dans l'eau et dans les solvants organiques usuels tels que l'oxyde de diéthyle, le dichlorométhane ; toutefois, en solution aqueuse, il s'hydrolyse.

**Microanalyse**

| | | C % | H % |
|---|---|---|---|
| $C_8H_{12}O_4$ | calculés | 55,80 | 7,03 |
| | trouvés | 55,8 | 7,0 |

Analyse physique RMN$^{13}$C (CDCl$_3$) à 50,4 MHz

$$COOH \quad : \quad 170,8 \text{ ppm}$$
$$=CH- \quad : \quad 133,25 \text{ ppm}$$
$$CH_2= \quad : \quad 116,3 \text{ ppm}$$
$$\begin{matrix} O \\ \quad CH- \\ O \end{matrix} \quad : \quad 95,8 \text{ ppm}$$
$$CH_2-O \quad : \quad 67,8 \text{ ppm}$$

## Exemple 4

On dissout à la température ambiante 230 g (3,2 moles) d'acide acrylique dans 150 g d'eau contenant 129 g (2,3 moles) d'hydroxyde de potassium, puis dans cette solution, on introduit :
- 10 g d'une solution aqueuse d'acide diéthylènetriaminopentaacétique à 9,6 g/l,
- 467 mg de persulfate de sodium dissous dans 5 g d'eau,
- 115 mg (0,59 mmole) de diallyloxyacétate de sodium dissous dans 5 g d'eau,

- 104,4 g d'eau.

On obtient ainsi 634 g d'une solution aqueuse désignée A.

Dans un réacteur de polymérisation, on chauffe au reflux, sous agitation, pendant 30 minutes, en atmosphère inerte :

- 634 g de cyclohexane,
- 3,5 g d'éthylcellulose contenant de 48 a 49,5 % de groupements éthoxylés et présentant à 25°C une viscosité de 200 mPa.s,

puis on introduit en 90 minutes, sous agitation et en atmosphère inerte, la solution A. L'introduction terminée, on poursuit le reflux pendant 1 heure, puis on élimine 240 g d'eau par distillation azéotropique et enfin, on refroidit la suspension obtenue à la température ambiante. On filtre le polymère cherché puis on le sèche à poids constant à 80°C. On obtient ainsi 332 g de polymère acide acrylique-acrylate de sodium réticulé présentant les caractéristiques suivantes :

- capacité d'absorption d'eau : 213 g/g,
- capacité d'absorption d'une solution aqueuse saline à 9 g/l : 40,5 g/g,
- taux d'extractibles : 2,6 %

## Exemple 5

On prépare une solution aqueuse contenant :

- 366 g d'eau permutée,
- 52 g (0,93 mole) d'hydroxyde de potassium,
- 0,8 g (4,1 mmoles) de diallyloxyacétate de sodium,
- 80 g (1,11 mole) d'acide acrylique,
- 57 mg (0,11 mmole) de diéthylènetriaminopentaacétate de sodium.

Dans cette solution soigneusement désoxygénée, on introduit sous agitation, à 40°C :

- 160 mg (6,7 mmoles) de persulfate de sodium dissous dans 2 g d'eau,
- 120 mg (6,3 mmoles) de métabisulfite de sodium dissous dans 2 g d'eau.

Après un léger temps de latence, la polymérisation démarre et le milieu réactionnel se transforme en un gel avec un échauffement notable. Lorsque la température du milieu réactionnel est revenue à la température ambiante, le gel polymère obtenu est séché 16 heures à 40°C, puis 10 heures à 100°C. On obtient ainsi, après broyage, une poudre blanche constituée par un copolymère acide acrylique-acrylate de potassium réticulé présentant les caractéristiques suivantes :

- extrait sec : 93,8, %
- capacité d'absorption d'eau : 113 g/g,
- capacité d'absorption d'une solution aqueuse à 9 g/l de chlorure de sodium : 28,8 g.

## Revendications

1. L'acide diallyloxyacétique et ses sels d'addition avec les bases minérales, à l'exception du sel de sodium.

2. L'acide diallyloxyacétique.

3. Le diallyloxyacétate de potassium.

4. Le diallyloxyacétate d'ammonium.

5. Procédé de préparation de l'acide diallyloxyacétique et de ses d'addition avec les bases minérales, à l'exception du sel de sodium, caractérisé en ce que l'on effectue une hydrolyse alcaline du diallyloxyacétate d'alkyle pour obtenir le sel basique correspondant que soit l'on isole, soit si désiré l'on acidifie en acide diallyloxyacétique que l'on isole ou si désiré, salifie en sel d'ammonium.

6. Procédé selon la revendication 5, caractérisé en ce que le diallyloxyacétate d'allyle est obtenu en faisant réagir l'acide glyoxylique avec de l'alcool allylique.

7. Application de l'acide diallyloxyacétique comme agent réticulant.

8. Application du diallyloxyacétate de sodium, de potassium, de lithium ou d'ammonium comme agent réticulant.

**Patentansprüche**

1. Diallyloxyessigsäure und ihre Additionssalze mit mineralischen Basen, mit Ausnahme des Natriumsalzes.

2. Diallyloxyessigsäure.

3. Kaliumdiallyloxyacetat.

4. Ammoniumdiallyloxyacetat.

5. Verfahren zur Herstellung von Diallyloxyessigsäure und ihren Additionssalzen mit mineralischen Basen, mit Ausnahme des Natriumsalzes, dadurch gekennzeichnet, daß eine basische Hydrolyse eines Diallyloxyessigsäurealkylester durchgeführt wird, wodurch das entsprechende basische Salz erhalten wird, das entweder isoliert, oder , wenn gewünscht, durch Ansäuern in die Diallyloxyessigsäure überführt wird, die isoliert, oder, wenn gewünscht, durch Versalzen in das Ammoniumsalz überführt wird.

6. Verfahren nach Anspruch 5, dadurch gekennzeichnet, daß der Diallyloxyessigsäureallylester dadurch erhalten wird, daß Glyoxylsäure mit Allylalkohol zur Reaktion gebracht wird.

7. Verwendung der Diallyloxyessigsäure als Vernetzungsmittel.

8. Verwendung von Natrium-, Kalium-, Lithium- oder Ammoniumdiallyloxyacetat als Vernetzungsmittel.

**Claims**

1. Diallyloxyacetic acid and its addition salts with mineral bases, with the exception of the sodium salt.

2. Diallyloxyacetic acid.

3. Potassium diallyloxyacetate.

4. Ammonium diallyloxyacetate.

5. Preparation process for diallyloxyacetic acid and its addition salts with mineral bases, with the exception of the sodium salt, characterised in that an alkaline hydrolysis of alkyl diallyloxyacetate is carried out so as to obtain the corresponding basic salt, which is either isolated, or if desired is acidified to diallyloxyacetic acid which is either isolated, or if desired, salified to the ammonium salt.

6. Process according to claim 5, characterised in that allyl diallyloxyacetate is obtained by reacting glyoxylic acid with allyl alcohol.

7. Application of diallyloxyacetic acid as a crosslinking agent.

8. Application of sodium, potassium, lithium or ammonium diallyloxyacetate as a cross-linking agent.